# EUROPEAN PATENT SPECIFICATION

(11) **EP 0 353 087 B1**
(45) Date of publication and mention of the grant of the patent: **03.04.1996**
(21) Application number: 89307691.9
(22) Date of filing: 28.07.1989
(51) Int. Cl.: A61B 17/22

(54) **Atherectomy device**
Atherektomievorrichtung
Dispositif d'athérectomie

(30) Priority: 29.07.1988 US 225880; 22.03.1989 US 326967
(43) Date of publication of application: 31.01.1990
(73) Proprietor: Shiber, Samuel, Atkinson, NH 03811 (US)
(72) Inventor: Shiber, Samuel, Atkinson, NH 03811 (US)
(74) Representative: Read, Matthew Charles

(56) References cited:
- EP-A- 0 163 502
- EP-A- 0 254 414
- EP-A- 0 347 098
- GB-A- 1 169 419
- D3=: Pupp: Vakuumtechnik
- B. Braun Melsungen AG - Product Literature

## Description

This invention relates to an atherectomy system for cutting, ingesting and removing an obstruction from within a patient's artery.

With age a large percentage of the population developes arterial obstructions formed by fats, fibrous material and calcified deposits, resulting in a diminished blood circulation. The disturbance to blood flow which these obstructions cause may induce blood clots which further diminish or block the blood flow. When this process occurs in the coronary arteries it is referred to as a heart attack. Presently such obstructions are circumvented by surgically grafting a bypass or they are treated by a catheter equipped with a balloon which is inserted through the arterial system, over a flexible guide-wire, into the obstruction and then inflated to expand the obstruction's lumen (angioplasty). Problems with this treatment are that it injures the arterial wall creating a rough lumen and in certain cases it is ineffective. Further, angioplasty does not remove the obstruction material out of the arterial system, therefore in a case of a heart attack, immediate angioplasty carries the risk of dislodging the blood clot and allowing it to move down stream creating additional blockages.

Prior art document EPA 0254414 discloses a rotary catheter insertable into a patients blood vessel for cutting ingesting and removing an obstruction therein, the device including suction means to assist the flexible rotary catheter in ingesting the obstruction material.

An objective of the invention is to provide an atherectomy catheter rotatable over a flexible guide wire, equipped with a rotary cutting means at its distal end, that would cut and ingest the obstruction material including blood clots if present, create a smooth lumen and not crack the arterial wall.

A further objective of the present invention is to provide suction means which will assist the flexible rotary catheter in ingesting the obstruction material.

In the present invention therefore the suction means is a positive displacement pump means.

Preferably, the suction means is self regulating and is used to automatically increase and decrease the suction in response to the presence or the absence, respectively, of obstruction material in the flexible rotary catheter. Thereby, such suction means reduces the amount of blood removed from the patient and prevents the arterial wall from collapsing in the absence of obstruction material.

Another objective of the invention is to provide a system that would lend itself to be produceable in diameters down to around 1mm (millimeter) and a length of up to around a meter, to be able to reach and enter small and remote arteries.

Preferably, the operation of the atherectomy system would resemble the operation of present catheter systems, so that existing skills of the medical staff can be utilised. These and other objectives of the invention will become apparent from the following discussion and the accompanying drawings.

In order that the invention may be more fully understood an illustrative embodiment will now be described with reference to the accompanying drawings wherein:
Figure 1 generally shows a cross sectional view of an atherectomy system according to the present invention;
Figure 2 shows a distal end of the atherectomy system.
Figure 3 shows a cross sectional view of the atherectomy system along a line 3-3 marked on Figure 1.

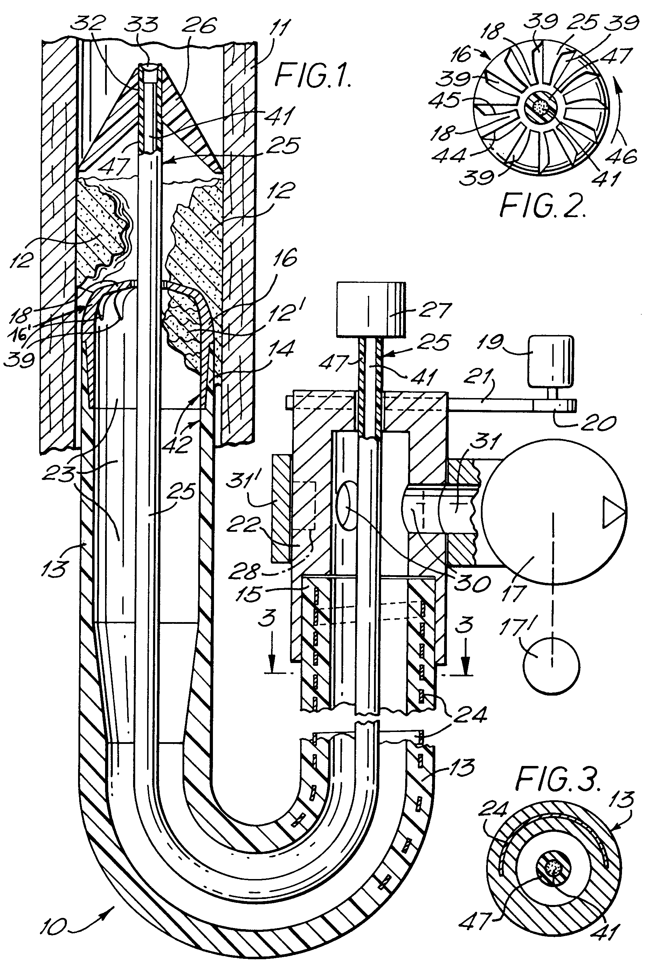

Figure 1 shows the atherectomy system 10 for cutting, ingesting and removing an obstruction 12 from within a patients vessel, an artery 11. As shown in Figure 1, the atherectomy system comprises several elongated parts in a nested relationship, and their ends shall be referred to as "distal", meaning the end which goes into the artery and "proximal", meaning the other end. Therefore, "distal direction" or the term "distally" shall indicate a general direction from the proximal end to the distal end, and "proximal direction" or "proximally" shall refer to an opposite direction.

The atherectomy system comprises:
A flexible guide-wire 25 which is insertable into the artery. Optionally, the flexible guide-wire is equipped at its distal end 32 with a distal barrier means in the form of a flexible collapsible umbrella 26 to counter distal movement of surrounding obstruction material while the blade cuts the obstruction material. The flexible guide-wire may also contain an optical fibre bundle 41 in a plastic jacket 47 and a lens 33 at its distal tip. An imaging unit and/or laser gun 27 may be optically coupled to the proximal end of the optical fibre bundle for analysing the inside of the artery and/or opening, respectively, a pilot passage for the distal tip of the flexible guide-wire to pass through in a case of complete arterial blockage.

A flexible rotary-catheter 13 is rotatably disposed and slidable over the flexible guide-wire.

A stainless steel hollow blade 16 is fitted into a distal end 14 of the flexible rotary-catheter. The blade has teeth 18 on its periphery which are bent inwardly, toward the center of the blade, to ease insertion through the arteries and to reduce the chance of cutting the wall of the artery during the insertion and cutting operation. A front edge 44 of the teeth is sharpened to cut the obstruction material to pieces 12' which pass into a continuous passage 23 through spaces 39 between the teeth while the blade rotates forward in a direction of arrow 46 (note Figure 2). A back side of the teeth 45 is dull to allow a backwards rotation while manipulating and advancing the flexible rotary-catheter through the arterial system towards the obstruction with a reduced risk of injuring the arterial wall. The blade has an outer wall 16' which slidingly and rotatably bears against the artery spreading the contact force on a relatively large area and thereby minimising the damage to the artery. A rotating inner-wall 42 is formed by the inside surface of the flexible rotary-catheter.

The continuous passage 23 is defined between the rotating inner-wall and the flexible guide-wire, and the relative motion between the flexible rotary catheter and the flexible guide-wire mechanically acts on the ingested obstruction material in the continuous passage and enables it to move towards the proximal end 15 of the flexible rotary-catheter and make room for additional cut material.

Coupling means affixed to the proximal end of the flexible rotary-catheter in the form of a hub 22 is frictionally engaged with a flat belt 21 which couples the flexible rotary-catheter to a rotating means in the form of a motor 19 having a pulley 20. The proximal end of the flexible guide-wire slidingly and rotatably extends through the hub.

Suction can be applied to the proximal end of the flexible rotary catheter by, preferably, a positive displacement type suction pump 17, driven by a motor 17′. The suction is applied through ports 30 which alternately communicate with a port 31 formed in a sleeve 31′, as the hub rotates in the sleeve 31′. Alternatively, a groove 28 (shown in phantom lines) can provide continuous communication between the continuous passage 23 and the port 31. The suction cooperates with the mechanical action taking place in the continuous passage to move the cut obstruction material 12′ proximally. A positive displacement pump such as a piston pump or a peristalic pump tends to self regulate the evacuation process. The amount of blood removed is limited by the volumn that is positively displaced by the pump. When only blood is present, and since blood flows relatively easily, the negative pressure in the continuous passage will drop. As obstruction material enters the continuous passage the negative pressure rises and pulls the cut material proximally. The level of negative pressure can be limited by a relief valve in the pump. The action of the pump can be synchronised with the actual cutting action of the blade 16, or otherwise selectively controlled to avoid excessive blood removal.

Torque generated by the motor is partially dissipated by frictional losses along the flexible rotary-catheter, therefore, the flexible rotary-catheter can be manufactured with an increased wall thickness and increased torque carrying capacity at the vicinity of its proximal end compared with the same at its distal end (note Figure 1), and the wall can be reinforced by a spiral means in the form of metal ribbon 24 (note Figures 1 and 3). The atherectomy system can be manufactured in different diameters and lengths depending on the size and site of the artery that it is intended for and on whether the system is to be used percutaneously (that is through the skin) or intraoperatively (that is when the artery is surgically exposed for inserting the system).

A process for removing an obstruction from an artery with an atherectomy system, comprises the following steps:

Conventionally inserting into an artery, into an obstruction, a flexible guide-wire.

Advancing over the flexible guide-wire a blade located at a distal end of an atherectomy catheter.

Advancing the blade to the obstruction and cutting the obstruction. During the operation the flexible guide-wire and the flexible introducer sleeve (if present) are prevented from being rotationally dragged by the blade. Fluid can be delivered to the obstruction site through the flexible sleeve, around the atherectomy catheter. Such fluid can lubricate and cool the cutting process and provide a medium for flushing particles of obstruction material into the atherectomy catheter, especially in conjunction with suction, preferably applied to the proximal end of the atherectomy catheter by a positive displacement pump means. The fluid may be radio-opaque to assist x-raying the process. Prior to cutting, fluid can also be delivered through the atherectomy catheter.

Removing the catheter containing the cut obstruction material out of the artery.

The sequence of insertion of the components into the artery may vary depending on the nature and location of the obstruction and the preferences of the medical staff. Additional steps may be added to assist the process. For example, a standard guiding catheter, which is either straight or pre-bent, may be inserted into the artery to assist in bringing the flexible guide-wire and the atherectomy catheter to the obstruction site.

When an arterial obstruction is further blocked by a fresh blood clot, as is often the case in a heart attack, the flexible guide-wire can usually be inserted through the fresh clot and the atherectomy system, preferably while employing suction, can be used to clear the clot in order to restore blood flow through the artery and alleviate the acute heart attack. Then the system can be utilised to cut the underlying atherosclerotic obstruction providing a long term correction to the condition that induced the attack.

## Claims

1. An atherectomy system for cutting, ingesting and removing an obstruction from within a patient's artery, comprising a flexible guidewire (25) insertable into said artery, a flexible rotary-catheter (13) rotatably disposed and slidable over the flexible guidewire (25), a blade (16) forming a distal end of the flexible rotary-catheter (13) having at least one tooth (18) on its periphery which is bent inwardly, a continuous passage (23) surrounding the flexible guidewire (25) for ingesting the cut obstruction material, the continuous passage (23) being defined between the flexible rotary-catheter (13) and the flexible guidewire (25), coupling means (22) at the proximal end of the flexible rotary-catheter (25) for coupling it to rotating means (19) and suction means (17) connected to said continuous passage (23) to pull cut obstruction material proximally characterised by said suction means (17) is a positive displacement pump means.

2. An atherectomy system as claimed in claim 1 characterised in that the thickness of the wall of the flexible rotary-catheter (25) at the vicinity of its proximal end is greater than that at its distal end to provide a carrying capacity in the vicinity of its proximal end.

3. An atherectomy system as claimed in claim 1 or claim 2 characterised in that the wall (13) of the flexible rotary-catheter (25) is reinforced with a spiral member (24).

4. An atherectomy system as claimed in claim 3 characterised in that the spiral member (24) is made of metal.

5. An atherectomy system as claimed in claim 3 or claim 4 characterised in that the spiral member (24) is made of a flat metal ribbon.

6. An atherectomy system as claimed in any preceding claim characterised in that the flexible guidewire (25) has distal barrier means (26) to counter distal movement of surrounding obstruction material while the blade (16) cuts the obstruction material.

7. An atherectomy system as claimed in any preceding claim characterised in that the flexible guide wire (25) contains an optical fibre (41).

8. An atherectomy system as claimed in any preceding claim characterised in that the displacement pump (17) is a piston pump.

9. An atherectomy system as claimed in any preceding claim characterised in that the displacement pump is a peristaltic pump.

## Patentansprüche

1. Atherektomie-System zum Zerschneiden, zur Aufnahme und Entfernung einer Obstruktion aus dem Innern einer Arterie eines Patienten, umfassend einen flexiblen Führungsdraht (25), der in diese Arterie einführbar ist, einen flexiblen Drehkatheter (13), der über dem flexiblen Führungsdraht (25) rotierbar angeordnet und verschiebbar ist, eine Klinge (16), die ein distales Ende des flexiblen Drehkatheters (13) bildet und an ihrem Umfang mindestens einen Zahn (18) aufweist, der nach innen gebogen ist, einen den flexiblen Führungsdraht (25) umgebenden durchgehenden Kanal (23) zur Aufnahme des herausgeschnittenen Obstruktionsmaterials, wobei der durchgehende Kanal (23) zwischen dem flexiblen Drehkatheter (13) und dem flexiblen Führungsdraht (25) definiert ist, Kupplungsmittel (22) am proximalen Ende des flexiblen Drehkatheters (25) zu seiner Ankupplung an Drehmittel (19) sowie Saugmittel (17), die mit dem durchgehenden Kanal (23) verbunden sind, um das Obstruktionsmaterial in proximale Richtung zu ziehen, dadurch gekennzeichnet, daß das Saugmittel (17) ein Verdrängerpumpmittel ist.

2. Atherektomie-System nach Anspruch 1, dadurch gekennzeichnet, daß die Dicke der Wand des flexiblen Drehkatheters (25) in der Nähe seines proximalen Endes größer ist als die an seinem distalen Ende, um ein Belastbarkeit bzw. Leistungsvermögen in der Nähe seines proximalen Endes zu schaffen.

3. Atherektomie-System nach Anspruch 1 oder Anspruch 2, dadurch gekennzeichnet, daß die Wand (13) des flexiblen Drehkatheters (25) mit einem schraubenlinienförmig gewundenen Element (24) verstärkt ist.

4. Atherektomie-System nach Anspruch 3, dadurch gekennzeichnet, daß das schraubenlinienförmig gewundene Element (24) aus Metall ist.

5. Atherektomie-System nach Anspruch 3 oder Anspruch 4, dadurch gekennzeichnet, daß das schraubenlinienförmig gewundene Element (24) aus einem flachen Metallband hergestellt ist.

6. Atherektomie-System nach irgendeinem der vorhergehenden Ansprüche, dadurch gekennzeichnet, daß der flexible Führungsdraht (25) ein distales hindernisbildendes Mittel (26) aufweist, um einer distalen Bewegung von umgebendem Obstruktionsmaterial entgegenzuwirken, während die Klinge (16) das Obstruktionsmaterial zerschneidet.

7. Atherektomie-System nach irgendeinem der vorhergehenden Ansprüche, dadurch gekennzeichnet, daß der flexible Führungsdraht (25) einen Lichtleiter (41) enthält.

8. Atherektomie-System nach irgendeinem der vorhergehenden Ansprüche, dadurch gekennzeichnet, daß die Verdrängerpumpe (17) eine Kolbenpumpe ist.

9. Atherektomie-System nach irgendeinem der vorhergehenden Ansprüche, dadurch gekennzeichnet, daß die Verdrängerpumpe eine peristaltische Pumpe ist.

## Revendications

1. Système d'athérectomie pour découper, ingérer et extraire une obstruction hors d'une artère d'un patient, comprenant un fil de guidage souple (25) pouvant être inséré dans cette artère, un cathéter rotatif souple (13), disposé rotatif et coulissant sur le fil de guidage souple (25), une lame constituant une extrémité distale du cathéter rotatif souple (13), ayant sur sa périphérie au moins une dent (18) recourbée vers l'intérieur, un passage continu (23) entourant le fil de guidage souple (25) pour ingérer la matière d'obstruction découpée, le passage continu (23) étant créé entre le cathéter rotatif souple (13) et le fil de guidage souple (25), des moyens d'accouplement (22) à l'extrémité proximale du cathéter rotatif souple (25) pour l'accoupler à des moyens rotatifs (19) et à des moyens d'aspiration (17) connectés dudit passage continu (23) en vue d'attirer proximalement la matière d'obstruction découpée, caractérisé par le fait que lesdits moyens d'aspiration (17) consistent en une pompe à déplacement positif.

2. Système d'athérectomie selon la revendication 1, caractérisé en ce que l'épaisseur de la paroi du cathéter rotatif souple (25) au voisinage de son extrémité proximale est supérieure à celle de son extrémité distale, en vue de créer une capacité de transfert au voisinage de son extrémité proximale.

3. Système d'athérectomie selon la revendication 1 ou la revendication 2, caractérisé en ce que la paroi (13) du cathéter rotatif souple est renforcée par un élément en spirale.

4. Système d'athérectomie selon la revendication 3, caractérisé en ce que l'élément en spirale (24) est réalisé en métal.

5. Système d'athérectomie selon la revendication 3 ou la revendication 4, caractérisé en ce que l'élément en spirale (24) est réalisé en ruban métallique plat.

6. Système d'athérectomie selon l'une quelconque des revendications précédentes, caractérisé en ce que le fil de guidage souple (25) comporte une barrière distale (26) pour s'opposer au déplacement distal de la matière d'obstruction qui l'entoure tandis que la lame (16) découpe la matière d'obstruction.

7. Système d'athérectomie selon l'une quelconque des revendications précédentes, caractérisé en ce que le fil de guidage souple (25) contient une fibre optique (41).

8. Système d'athérectomie selon l'une quelconque des revendications précédentes, caractérisé en ce que la pompe à déplacement (17) est une pompe à piston.

9. Système d'athérectomie selon l'une quelconque des revendications précédentes, caractérisé en ce que la pompe à déplacement est une pompe péristaltique.
